# EUROPEAN PATENT APPLICATION

(11) **EP 2 332 607 A1**
(43) Date of publication of application: **15.06.2011**
(21) Application number: 09394034.4
(22) Date of filing: 14.12.2009
(51) Int. Cl.: A61M 25/00

(54) **A urethral catheter**

(71) Applicant: The Royal College of Surgeons in Ireland, Dublin 2 (GB)
(72) Inventor: Guillermo Rodriquez Navas, Jose , Technology, Dublin 2 (IE)
(74) Representative: O'Brien, John Augustine

(57) **Abstract**

A urethral catheter 1 for drainage of urine from the bladder comprises a distal tip 2, a proximal part 3, and a flexible tube 5 extending between proximal part 3 and the distal tip 2. The tube 5 has a distal anchor 6 and comprises a plurality of drainage orifices 10 which are spaced-apart along a distal section of the tube 5. The catheter 1 also has an irrigation lumen 50 with a plurality of irrigation orifices 55 which are spaced-apart along a distal section of the irrigation tube. The irrigation orifices 55 ensure that the drainage orifices 10 do not became blocked.

## Description

### Introduction

This invention relates to a urethral catheter.

There are many complications associated with urethral catheters in current use. The severity and impact differ from patient to patient. The complications/problems include the following:
1. Acute urethral rupture.
2. Urethritis.
3. Peri-urethral abscess.
4. Urethra-Cutaneous fistula.
5. Urethral stricture.
6. Bladder perforation.
7. Bleeding due to tissue trauma.
8. Paraphimosis.
9. Urethral Diverticula.
10. Urethral false passage.
11. Urinary tract infection.
12. Chronic bacterial cystitis.
13. Urinary incontinence after catheter withdrawal.
14. Catheter fracture.
15. Vesicorenal reflux.
16. Unintentional catheterisation of the ureter.
17. Rupture of the balloon of the catheter.
18. Failure of the balloon of the catheter to deflate for withdrawal of the catheter.
19. Blockage of the catheter.
20. Urine bypassing the catheter.
21. Urine pooling in the bladder or urine retention even in the presence of the catheter.
22. Catheter knotting in situ.
23. Catheter-related pain.
24. Difficulty of self-catheterisation.
25. Difficult catheterisation on patients with an enlarged prostate.
26. Difficult catheterisation on patients with urethral stenosis.
27. Lack of privacy or independence in empting the bladder.
28. Catheter associated with increased risk of bacteraemia and sepsis.
29. Embarassment for many patients unable to use underwear because of the indwelling catheterisation.
30. Discomfort for patients walking with the catheter in place.
31. Difficulties for the staff and the patient in transferring from a bed to a trolley, or a wheelchair when the patient has a urethral catheter connected to the urine bag.
32. Patients with indwelling catheter develop more often infections caused by highly resistant bacteria.
33. Catheters contribute to selection, resistance, spread and persistence of highly resistant bacteria.
34. Difficulty in patients sleeping with a urethral catheter connected to a urine bag.
35. Patients under an intermittent catheterisation regime often develop depression and low self esteem.
36. Patients under intermittent catheterisation need to carry many devices every time they go out of home; and then look for an hygienic toilet to perform self catheterisation, otherwise they cannot leave home or go to places without those facilities.

The invention is directed towards providing an improved urethral catheter which will address at least some of these issues.

### Statements of Invention

According to the invention there is provided a urethral catheter comprising:-
a distal tip;
a proximal port; and
a flexible tube extending between the distal tip and the proximal port,
the tube having:-
a distal anchoring section adjacent to the distal tip; and
at least one drainage orifice proximal of the distal anchoring section.

In one embodiment the distal tip comprises a guiding tip.

The distal tip may be stiffer than the flexible tube. In one case the distal tip comprises an extension of the flexible tube and a stiffening means for the distal tip.

In one embodiment the distal tip tapers distally.

The tube may comprise a plurality of spaced-apart drainage orifices. In one case the drainage orifices are spaced-apart along a distal section of the catheter. The orifices may be spaced-apart longitudinally along the distal section. There may be a first set of orifices and a second set of orifices which are directed differently to the first set of orifices. In one case the second set of orifices are diametrically opposed to the first set of orifices. The first set of orifices may be aligned with the second set of orifices.

In one embodiment there are at least 3 drainage orifices, at least 4 drainage orifices.

In one embodiment there are from 2 to 20 drainage orifices.

In one case there may be 14 drainage orifices.

In another case there may be 10 drainage orifices.

In one embodiment the distal anchoring section has a normally curvilinear shape. The distal anchoring section may have a delivery configuration which is relatively straight.

The distal anchoring section may be of a shape memory material. In one case the distal anchoring section is of the same material as that of the flexible tube.

In one embodiment the flexible tube is of a polymeric material. The flexible tube may be of a polyurethane material.

In one embodiment the urethral catheter comprises a flexible proximal portion adjacent to the proximal port.

The proximal portion may be more flexible than the main body of the catheter. In one case the proximal portion is of the same material as that of the catheter tube.

In another case the proximal portion is of a different material to that of the catheter tube.

The proximal portion may define the proximal port.

In one embodiment the urethral catheter comprises a cap for the proximal port. The cap may be removable.

In one embodiment the flexible tube has indicator marks or indicia thereon. The indicator marks may be provided adjacent a proximal end of the flexible tube.

In one embodiment the catheter comprises an irrigation lumen. The irrigation lumen may be defined by an irrigation tube. The irrigation tube may be located internally of the catheter flexible tube. In one case the inner irrigation tube extends from a proximal end to a location which is distal of the drainage orifice.

In one embodiment the irrigation tube comprises a plurality of outlets. The irrigation outlets may be located adjacent to the catheter tube drainage outlets. In one case the irrigation outlets are located distal of the most distal drainage outlet.

The irrigation outlets may be spaced-apart along a distal section of the irrigation tube.

In one case the irrigation tube is of the same material as that of the catheter tube.

The irrigation tube may comprise a proximal cap. The proximal cap may be removable.

The invention also provides a urethral catheter comprising:
a distal tip;
a proximal port, and
a flexible tube extending between the distal tip and the proximal port,
the tube having at least one drainage orifice proximal of the distal tip and wherein the catheter comprises an irrigation lumen having at least one outlet for an irrigation fluid.

The invention also provides a urethral catheter comprising:-
a distal tip;
a proximal port;
a flexible tube extending between the distal tip and the proximal port, and a distal anchor adjacent to the distal tip,
the distal anchor having a delivery configuration and a deployed configuration wherein the distal anchor is curvilinear in the deployed configuration and is relatively straight in the delivery configuration.

### Brief Description of the Drawings

The invention will be more clearly understood from the following description of an embodiment thereof, given by way of example only, with reference to the accompanying drawings, in which:
Fig. 1 is an isometric view of a urethral catheter according to the invention;
Fig. 2 is an elevational view of the catheter of Fig. 1;
Fig. 3 is a cross sectional view on the line III - III of Fig. 2;
Fig. 4 is an enlarged cross sectional view of a distal end of the catheter;
Fig. 5 is an enlarged cross sectional view of a distal section of the catheter;
Fig. 6 is a cross sectional view on the line VI -VI of Fig. 5;
Fig. 7 is a cross sectional view on the line VII - VII of Fig. 5;
Fig. 8 is a cross sectional view on the line VIII - VIII of Fig. 5;
Fig. 9 is an enlarged cross sectional view of a proximal end of the catheter;
Fig. 10 is an isometric view of another urethral catheter according to the invention;
Fig. 11 is an enlarged cross sectional view of the distal end of the catheter of Fig. 10;
Fig. 12 is an enlarged cross sectional view of the proximal end of the catheter of Fig. 10;
Fig. 13 is an isometric view of a further urethral catheter of the invention; and
Fig. 14 is an isometric view of a still further urethral catheter of the invention.

### Detailed Description

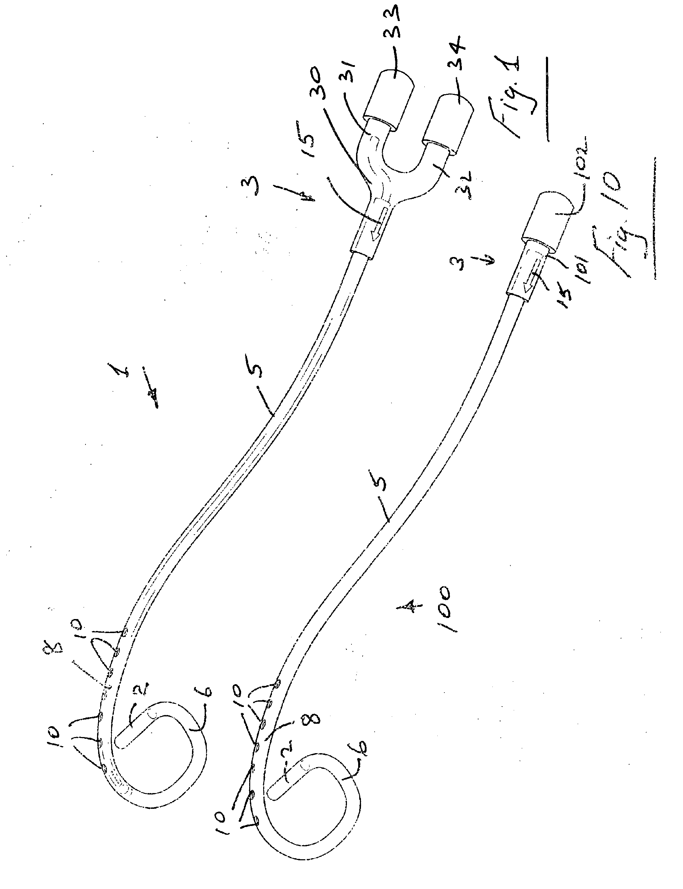

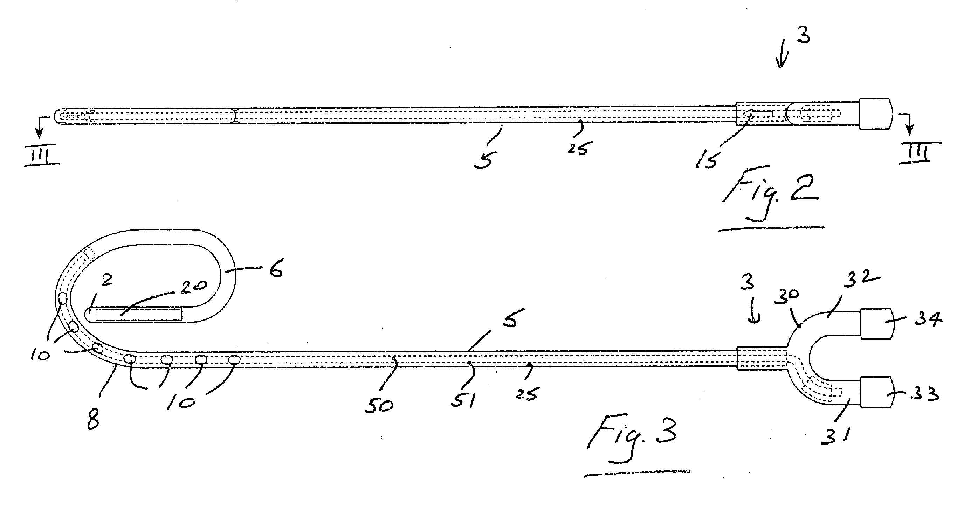

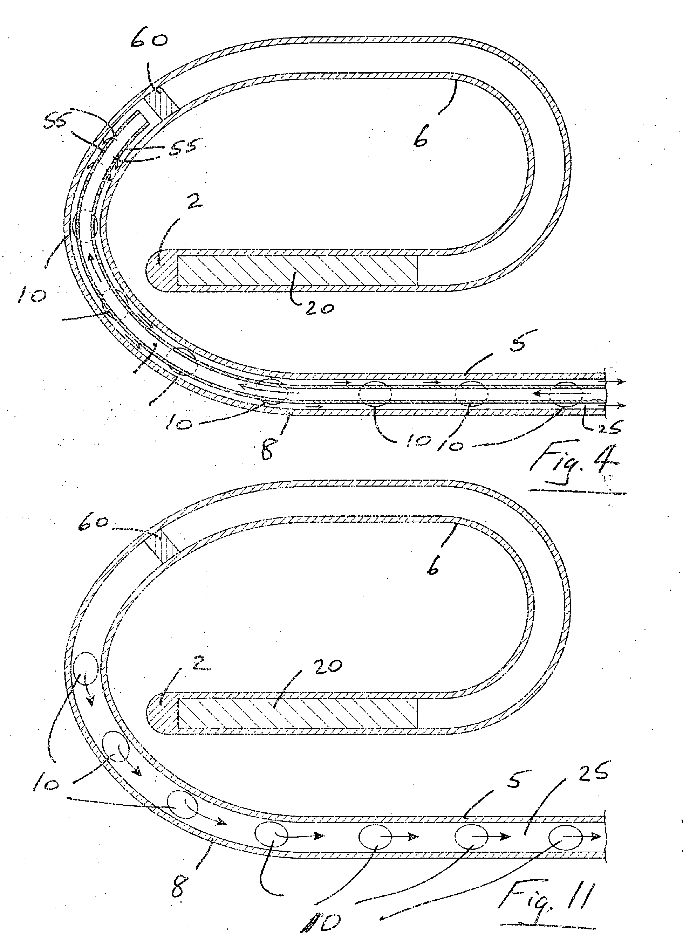

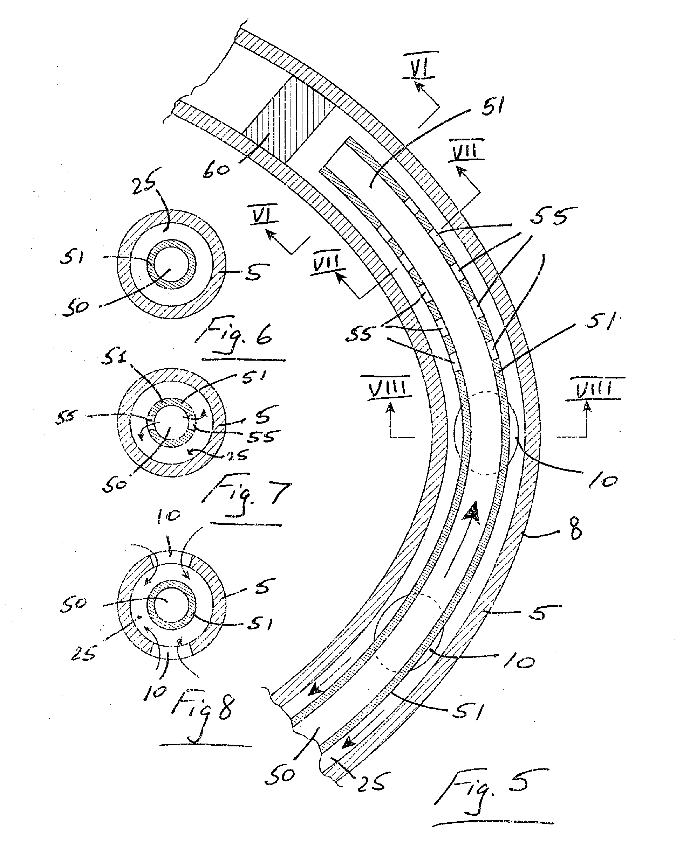

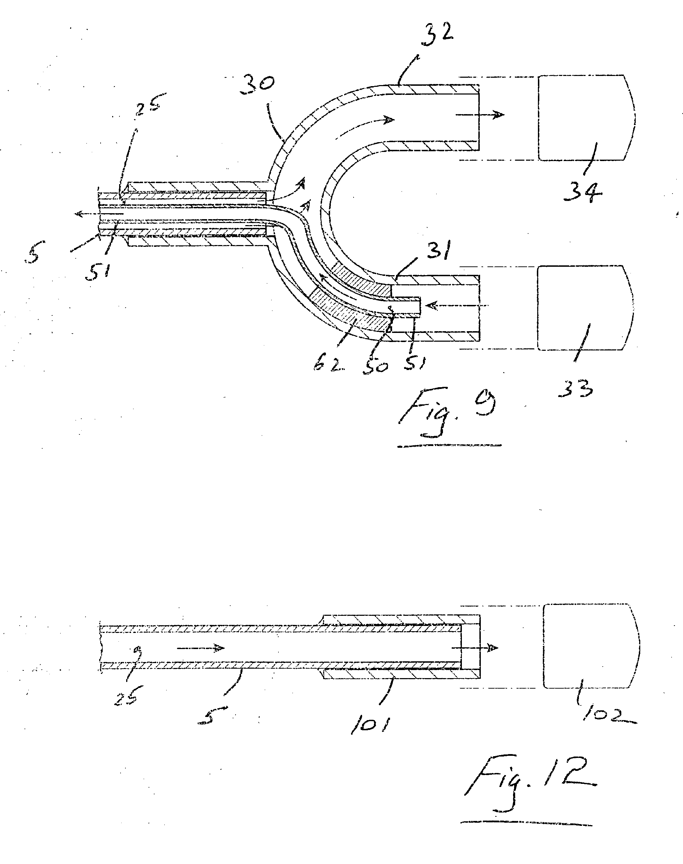

Referring to the drawings and initially to Figs. 1 to 9 there is illustrated a urethral catheter 1 for drainage of urine from the bladder, in this case a male bladder. The catheter 1 comprises a distal tip 2, a proximal part 3, and a flexible tube 5 extending between the proximal part 3 and the distal tip 2. The tube 5 has a distal anchor 6 and comprises at least one drainage orifice 10 proximal of the distal anchor 6. In this case there are 14 drainage orifices 10 which are arranged in two groups of 7 each which are spaced-apart along a distal section 8 of the tube 5 on opposite sides of the catheter tube 5. There is an indicia or mark, in this case in the form of an arrow 15, at the proximal end of the catheter. The arrow 15 is used to indicate to the user / clinician the orientation of the catheter when in situ in the bladder. The optimum orientation is when the arrow 15 is uppermost. In this orientation the orifices 10 are arranged on diametrically opposite sides of the tube for optimum drainage of urine from the bladder.

In this case the distal anchor 6 comprises a distal section of the tube 5 which is formed to have a normally curvilinear shape as illustrated so that on deployment in the bladder it will assume the curvilinear shape and anchor the catheter in situ. The distal anchor 6 is flexible to ensure a generally straight configuration in a delivery configuration to aid delivery and deployment. A distal most section of the catheter has a filler 20 which may be of a soft polyurethane material. The filler 20 assists in partially stiffening the distal end of the catheter to facilitate delivery by providing additional pushability to the catheter so that it can be readily located in a desired position in the bladder. This feature is particularly useful in navigating through the narrowed entry passageway to the bladder in the region of the prostrate of a male patient. Typically the filler 20 extends for a length of about 35mm. The distal end 2 of the catheter is tapered distally in this case in a dome shape to facilitate atraumatic delivery of the catheter.

The catheter 5 defines a fluid drainage lumen 25 through which fluid entering through the orifices 10 is drained. The fluid flows to the proximal end 3 of the tube. In this case the proximal end 3 of the catheter is fitted with a leur 30 of a soft and flexible material such as plastisol. In this case the leur 30 is a Y connector having two legs 31, 32 both of which are fitted with removable caps 33, 34. The caps 33, 34 are of a soft material and are a push fit onto the end of the legs 31, 32 of the leur. There may be features such as barbs on the inside of the caps 33, 34 to facilitate push fitting and ease of removal. In this case fluid drains from the catheter 5 through the drainage outlet leg 32 of the leur 30.

In this particular embodiment of the invention the catheter 1 also comprises an irrigation lumen 50 extending from the second leg 31 of the leur 30 to a distal end which in this case is distal of the drainage orifices 10 and proximal of the distal anchor 6 of the catheter. The irrigation lumen is in this instance defined by an irrigation tube 51 which extends through the catheter shaft 5. At the distal end of the irrigation tube 50 there are a plurality of irrigation orifices 55 which are spaced-apart along a distal section of the irrigation tube 50. It will be noted that all of the irrigation outlet orifices 55 are located distal of the most distal drainage outlet 10 of the catheter 5. The function of the irrigation is to ensure that the drainage orifices 10 do not become blocked. The flow of irrigation fluid exiting slowly through the irrigation outlets 55 provides a flow of irrigation fluid from the distal end that passes along the inside of the drainage orifices 10 in laminar flow and promotes flow of fluid through the drainage orifices 10 without blocking.

The irrigation tube 51 is bonded to the catheter shaft at the distal end thereof which is proximal of the anchor section 6. The catheter shaft is occluded, for example by a plug 60 in the region of the distal bond site for the irrigation tube 51 to prevent the flow of irrigation fluid into the anchor region 6. At the proximal end, the irrigation tube 51 is bonded to the leur leg 31 using a bonding block 62.

Referring now to Figs. 10 to 12 there is illustrated another urethral catheter 100 according to the invention which is similar to the catheter described with reference to Figs. 1 to 9 and like parts are assigned the same reference numerals. In this case the catheter is for simple drainage of the bladder and there is no irrigation tube. The proximal end of the catheter 100 is bonded to a soft plastisol end piece 101 which is covered by a removable cap 102.

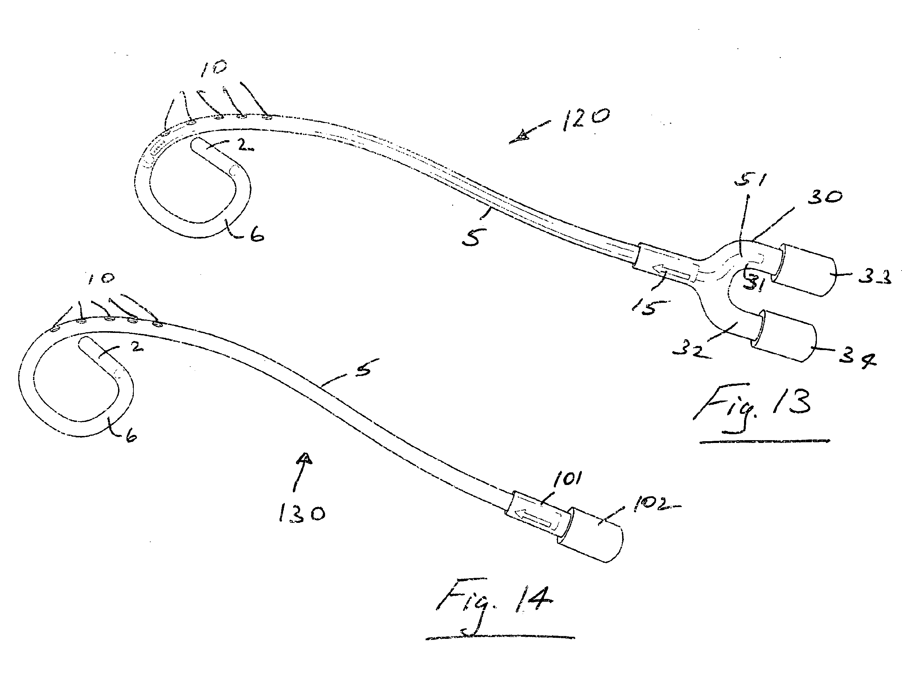

Referring to Fig. 13 there is illustrated another catheter 120 according to the invention which is of shorter length than the catheter of Figs. 1 to 9. Otherwise, however, the catheter 120 has similar features to the catheter of Figs. 1 to 9 and like parts are assigned the same reference numerals. The catheter according to this embodiment of the invention is particularly suitable for use with female patients.

Referring to Fig. 14 there is illustrated another catheter 130 according to the invention which is similar to the catheter described above with reference to Figs. 10 to 12 and like parts are assigned the same reference numerals. The catheter 130 is of shorter length than the catheter of Figs. 10 to 12 and is useful particularly for female patients.

Some advantages of the various features of the catheters of the invention are as follows:
Spiralled tip: the catheter uses a spiralled tip as an anchoring system to keep the catheter in place. This anchoring system is of an elastomeric substance with memory. It not only keeps the catheter in place, but is also designed to pass through any obstruction and avoid complications relating to the use of a balloon as an anchor. This spiralled tip and the guiding tip assist in overcoming obstructions. They facilitate catheterisation.

The absence of a balloon avoids complications such as rupture of the urethra, rupture of the balloon, and failure of the balloon to deflate.

Guiding tip: the guiding tip facilitates ease of catheterisation. The tip is typically about 2 cm long, narrow and straight. It is comfortable and easy to use to effect catheterisation either by the doctor or patient. The tip functions as a guide wire. The tip design avoids the risk of urethral damage during catheterisation and during withdrawal of the catheter. The guiding tip is tapered, narrow, and flexible and the orientation of the tip pointing up is in harmony with the anatomy of the urethra, especially the prostatic urethra in normal and in pathologic states.

Bladder orifices: the orifices for the bladder are designed to increase the drainage area of the catheter. It allows a quick and complete emptying of the bladder, avoiding pooling of the urine, which is a risk factor for urinary tract infection. The orifices reduce the shear forces between the urethra and the catheter during introduction and withdrawal. The bladder orifices the drainage area is large as there are multiple orifices. Thus, if any orifices become obstructed the remaining orifices retain drainage of the bladder. In addition, the bladder orifices accelerate drainage as they fall in the correct position to drain the bladder regardless of the position of the catheter during introduction. The bladder orifices also take advantage of the natural dynamic forces acting upon the bladder to enhance the drainage of the bladder.

The most proximal orifices of the catheter define prostate orifices that avoid urine bypassing and for drainage of the prostate area after surgery. If the internal sphincter is dysfunctional, then all urine that goes beyond it will be drained by these orifices. The prostate orifices diminishes the shear forces during introduction and withdraw. They also improve drainage of blood from the prostate area after surgery. The prostate orifices offer a direct drainage pathway for the bleeding avoiding the passage of large amounts of blood to the bladder with the subsequent clotting process inside the bladder, which could lead to an adverse event.

The catheter may have a proximal indicia or marks to provide a body scale. This scale has the important role of measuring the length of the urethra. After the first catheterisation the staff and the patient will know the length of the urethra and the correct position of the catheter. In subsequent catheterisation the staff will know exactly how much catheter they need to introduce for a perfect localization of it. This knowledge is important to avoid complications due to the catheter such as insufficient introduction or knotting of the catheter.

Flexible proximal funnel: This enhances patient comfort. It allows the patient to wear underwear or normal clothes with total comfort. It is also important in avoiding pain during catheter manipulation because there is no transmission of force backward. This avoids urethral complications such urethritis, urethral fistula and other.

Cap; the cap of the catheter has different functions. First, it is to close the catheter every time the patient needs to be transported, avoiding the risk of urine reflux from a collection bag. Second, it is more comfortable for the patient to walk or use the toilet with the catheter closed rather than the bag hanging. Third, it is more comfortable for the patient to sleep with the catheter closed. The cap also facilitates easier and more hygienic bag changing. It helps to avoid catheter-related urinary tract infections as it keeps the system closed. For patients under intermittent catheterisation the cap is particularly important. These patients can insert the catheter at home and then go out with total freedom without been concern of the availability of toilets and the hygienic conditions of it. Every time they need to empty their bladder while outside, they just go to any toilet, remove the cap, void the bladder and put on a new cap. When they are back in home, they remove the catheter. This is particularly advantageous for patients requiring intermittent catheterisation.

Irrigation system: this system enhances the drainage performance of the catheter. It produces a constant laminar flow inside the catheter. This constant washout of the lumen helps to remove any substance. When a viscous substance enters the catheter it is immediately diluted by the laminar flow of water. In the case of drainage of blood after surgery or trauma, the laminar flow avoids the contact of blood with the wall of the catheter, thus avoiding coagulation inside the catheter. The overall result is an enhanced drainage performance and avoidance of catheter blockage. At the same time, this system avoids the introduction of fluids inside the bladder with consequential associated risks. A further advantage of the irrigation system is that it avoids catheter encrustation by biofilms plus calcium and other substance that often cause catheter obstruction.

The invention is not limited to the embodiments hereinbefore described which may be varied in construction and detail.

## Claims

1. A urethral catheter comprising:-
a distal tip;
a proximal port; and
a flexible tube extending between the distal tip and the proximal port,
the tube having:-
a distal anchoring section adjacent to the distal tip; and
a plurality of drainage orifices spaced-apart along a distal section of the catheter which is proximal of the distal anchoring section.

2. A urethral catheter as claimed in claim 1 wherein the orifices are spaced - apart longitudinally along the distal section.

3. A urethral catheter as claimed in claim 2 comprising a first set of orifices and a second set of orifices which are directed differently to the first set of orifices.

4. A urethral catheter as claimed in claim 3 wherein the second set of orifices are diametrically opposed to the first set of orifices or are aligned with the second set of orifices.

5. A urethral catheter as claimed in any of claims 1 to 4 wherein the catheter comprises an irrigation lumen.

6. A urethral catheter as claimed in claim 5 wherein the irrigation lumen is defined by an irrigation tube.

7. A urethral catheter as claimed in claim 6 wherein the irrigation tube is located internally of the catheter flexible tube, preferably the inner irrigation tube extends from a proximal end to a location which is distal of the drainage orifice.

8. A urethral catheter as claimed in claim 6 or 7 wherein the irrigation tube comprises a plurality of outlets.

9. A urethral catheter as claimed in claim 8 wherein the irrigation outlets are located adjacent to the catheter tube drainage outlets, the irrigation outlets may be located distal of the most distal drainage outlet.

10. A urethral catheter as claimed in any of claims 6 to 9 wherein the irrigation outlets are spaced-apart along a distal section of the irrigation tube.

11. A urethral catheter as claimed in any of claims 6 to 10 wherein the irrigation tube comprises a proximal cap which may be removable.

12. A urethral catheter as claimed in any of claims 1 to 11 wherein the distal tip comprises a guiding tip, the distal tip may be stiffer than the flexible tube, the distal tip may comprise an extension of the flexible tube and a stiffening means for the distal tip, the distal tip may taper distally.

13. A urethral catheter as claimed in any of claims 1 to 12 wherein the distal anchoring section has a normally curvilinear shape, preferably the distal anchoring section has a delivery configuration which is relatively straight, the distal anchoring section may comprise a shape memory material.

14. A urethral catheter as claimed in any of claims 1 to 13 comprising a cap for the proximal port, the cap is preferably removable.

15. A urethral catheter as claimed in any of claims 1 to 14 wherein the flexible tube has indicator marks or indicia thereon.
